# EUROPEAN PATENT SPECIFICATION

(11) **EP 0 752 982 B1**
(45) Date of publication and mention of the grant of the patent: **14.10.1998**
(21) Application number: 95911321.8
(22) Date of filing: 09.03.1995
(51) Int. Cl.: C07C 233/75, C07C 235/84, C07C 251/48, C07C 255/57, A61K 31/165, A61K 31/275

(54) **BIPHENYL DERIVATIVES AS 5HT1D ANTAGONISTS**
BIPHENYL-DERIVATE ALS 5HT1D ANTAGONISTEN
DERIVES BIPHENYLES EN TANT QU'ANTAGONISTES DU 5HT1D

(30) Priority: 26.03.1994 GB 9406040; 26.03.1994 GB 9406041
(43) Date of publication of application: 15.01.1997
(73) Proprietor: SMITHKLINE BEECHAM PLC, Brentford, Middlesex TW8 9EP (GB)
(72) Inventor: GASTER, Laramie, Mary, SmithKline Beecham, Harlow Essex CM19 5AD (GB); WYMAN, Paul, Adrian SmithKline Beecham, Harlow Essex CM19 5AD (GB)
(74) Representative: Waters, David Martin, Dr.
(86) International application number: EP9500900
(87) International publication number: WO9526328

(56) References cited:
- EP-A- 0 496 378
- EP-A- 0 533 266
- CH-A- 622 005
- GB-A- 2 276 161
- US-A- 4 038 416

## Description

The present invention relates to novel amide derivatives, processes for their preparation, and pharmaceutical compositions containing them.

EPA 0 533 266/7/8 disclose a series of benzanilide derivatives which are said to possess 5HT_{1D} receptor antagonist activity. These compounds are said to be of use in the treatment of various CNS disorders.

A structurally distinct class of compounds have now been discovered and have been found to exhibit 5HT_{1D} antagonist activity. In a first aspect, the present invention therefore provides a compound of formula (I) or a salt thereof: 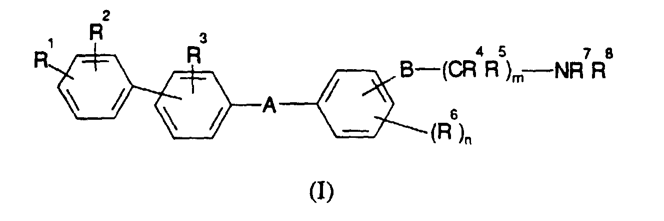 in which
R¹ is hydrogen, halogen, C₁₋₆alkyl, C₃₋₆cycloalkyl, COC₁₋₆alkyl, C₁₋₆alkoxy, hydroxy, hydroxyC₁₋₆alkyl, hydroxyC₁₋₆alkoxy, C₁₋₆alkoxyC₁₋₆alkoxy, acyl, nitro, trifluoromethyl, cyano, SR⁹, SOR⁹, SO₂R⁹, SO₂NR¹⁰R¹¹, CO₂R¹⁰, CONR¹⁰R¹¹, CO₂NR¹⁰R¹¹,CONR¹⁰(CH₂)ₚCO₂R¹¹, (CH₂)ₚNR¹⁰R¹¹, (CH₂)ₚCONR¹⁰R¹¹, (CH₂)ₚCO₂C₁₋₆alkyl, NR¹⁰R¹¹, NR¹⁰CO₂R¹¹, NR¹⁰CONR¹⁰R¹¹, CR¹⁰=NOR¹¹, CNR¹⁰=NOR¹¹, where R¹⁰ and R¹¹ are independently hydrogen or C₁₋₆alkyl and p is 1 to 3;
R² and R³ are independently hydrogen, halogen, C₁₋₆alkyl, C₃₋₆cycloalkyl, C₃₋₆cycloalkenyl, C₁₋₆alkoxy, acyl, aryl, acyloxy, hydroxy, nitro, trifluoromethyl, cyano, CO₂R¹⁰, CONR¹⁰R¹¹, NR¹⁰R¹¹ where R¹⁰ and R¹¹ are independently hydrogen or C₁₋₆alkyl;
R⁴ and R⁵ are independently hydrogen or C₁₋₆alkyl;
R⁶ is hydrogen, halogen, hydroxy, C₁₋₆alkyl or C₁₋₆alkoxy;
R⁷ and R⁸ are independently hydrogen, C₁₋₆alkyl, aralkyl, or together with the nitrogen atom to which they are attached form an optionally substituted 5-7-membered heterocyclic ring containing one or two heteroatoms selected from oxygen, nitrogen or sulphur;
A is CONH or NHCO;
B is oxygen, S(O)ₚ where p is 0, 1 or 2, NR¹² where R¹² is hydrogen,
C₁₋₆alkyl or phenylC₁₋₆alkyl, or B is CR⁴=CR⁵ where R⁴ and R⁵ are independently hydrogen or C₁₋₆alkyl;
m is 1 to 4; and
n is 1 or 2.

C₁₋₆alkyl groups, whether alone or as part of another group, may be straight chain or branched.

Suitably R¹ is hydrogen, halogen, C₁₋₆alkyl, C₃₋₆cycloalkyl, COC₁₋₆alkyl, C₁₋₆alkoxy, hydroxy, hydroxyC₁₋₆alkyl, hydroxyC₁₋₆alkoxy, C₁₋₆alkoxyC₁₋₆alkoxy, acyl, nitro, trifluoromethyl, cyano, SR⁹, SOR⁹, SO₂R⁹, SO₂NR¹⁰R¹¹, CO₂R¹⁰, CONR¹⁰R¹¹, CO₂NR¹⁰R¹¹, CONR¹⁰(CH₂)ₚCO₂R¹¹, (CH₂)ₚNR¹⁰R¹¹, (CH₂)ₚCONR¹⁰R¹¹, (CH₂)ₚCO₂C₁₋₆alkyl, NR¹⁰R¹¹, NR¹⁰CO₂R¹¹, NR¹⁰CONR¹⁰R¹¹, CR¹⁰=NOR¹¹, CNR¹⁰=NOR¹¹, where R¹⁰ and R¹¹ are independently hydrogen or C₁₋₆alkyl and p is 1 to 3.

Suitably R² and R³ are independently hydrogen, halogen, C₁₋₆alkyl, C₃₋₆cycloalkyl, C₃₋₆cycloalkenyl, C₁₋₆alkoxy, acyl, aryl, acyloxy, hydroxy, nitro, trifluoromethyl, cyano, CO₂R¹⁰, CONR¹⁰R¹¹, NR¹⁰R¹¹ where R¹⁰ and R¹¹ are independently hydrogen or C₁₋₆alkyl. Preferably R² is C₁₋₆alkyl, in particular methyl. Preferably R³ is hydrogen.

Suitably R⁴ and R⁵ are independently hydrogen or C₁₋₆alkyl.

Suitably R⁷ and R⁸ are independently hydrogen or C₁₋₆alkyl. Examples of R⁷ and R⁸ heterocyclic rings include morpholine, piperazine and piperidine. Optional substituents for such rings include C₁₋₆alkyl. Preferably R⁷ and R⁸ are both C₁₋₆alkyl, in particular methyl.

Suitably R⁶ is hydrogen, halogen, hydroxy, C₁₋₆alkyl or C₁₋₆alkoxy. Preferably R⁶ is C₁₋₆alkoxy such as methoxy.

Suitably A is CONH or NHCO. Preferably A is CONH.
Suitably B is oxygen, S(O)ₚ where p is 0, 1 or 2, NR¹² where R¹² is hydrogen, C₁₋₆alkyl or phenylC₁₋₆alkyl, or B is CR⁴=CR⁵ where R⁴ and R⁵ are independently hydrogen or C₁₋₆alkyl. Preferably B is oxygen.

Suitably m is 1 to 4, preferably m is 1 or 2

Suitably n is 1 or 2, preferably n is 1.

The groups -B(CR⁴R⁵)ₘNR⁷R⁸ and R⁶ can be attached to the phenyl ring at any suitable position. Preferably the group -B(CR⁴R⁵)ₘNR⁷R⁸ is meta to the amide linkage and the group R⁶ is para to the amide linkage. The groups R¹, R² and R³ can be attached at any suitable position.

Particularly preferred compounds of the invention include:
N-[3-(2-Dimethylaminoethoxy)4-methoxyphenyl]-4'-methoxycarbonyl-2'-methylbiphenyl-4-carboxamide,
N-[3-(2-Dimethylaminoethoxy)-4-methoxyphenyl]-2'-methyl-4'-N-methylcarboxamidobiphenyl-4-carboxamide,
N-[3-(2-Dimethylaminoethoxy)-4-methoxyphenyl]-2'-methyl-4'-N,N-dimethylcarboxamidobiphenyl-4-carboxamide,
N-[3-(2-Dimethylaminoethoxy)-4-methoxyphenyl]-2'-methyl-4'-carboxamidobiphenyl-4-carboxamide,
N-[3-(2-Dimethylaminoethoxy)4-methoxyphenyl]-2'-methyl4'-cyanobiphenyl-4-carboxamide,
N-[3-(2-Dimethylaminoethoxy)4-methoxyphenyl]-4'-acetyl-2'-methylbiphenyl-4-carboxamide,
N-[3-(2-Dimethylaminoethoxy)-4-methoxyphenyl]-4'-[1-(methoxyimino)ethyl]-2'-methylbiphenyl-4-carboxamide,
N-[3-(2-Dimethylaminoethoxy)-4-methoxyphenyl]-4'-[1-(hydroxyimino)ethyl]-2'-methylbiphenyl-4-carboxamide,
or a pharmaceutically acceptable salt thereof.

Preferred salts of the compounds of formula (I) are pharmaceutically acceptable salts. These include acid addition salts such as hydrochlorides, hydrobromides, phosphates, acetates, fumarates, maleates, tartrates, citrates, oxalates, methanesulphonates and p-toluenesulphonates.

Certain compounds of formula (I) are capable of existing in stereoisomeric forms. It will be understood that the invention encompasses all geometric and optical isomers of the compounds of formula (I) and the mixtures thereof including racemates. Tautomers and mixtures thereof also form an aspect of the invention.

In a further aspect the present invention provides a process for the preparation of a compound of formula (I) which comprises.
(a) reaction of a compound of formula (II): 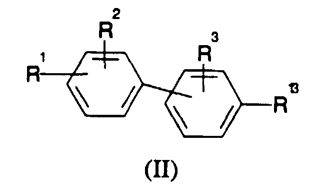 with a compound of formula (III): 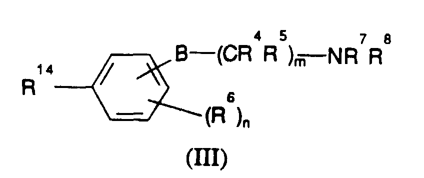
wherein B, m, n, R¹, R², R³, R⁴, R⁵, R⁶, R⁷ and R⁸ are as defined in formula (I) and R¹³ and R¹⁴ contain the appropriate functional group(s) necessary to form the A moiety; and optionally thereafter in any order:
- converting a compound of formula (I) into another compound of formula (I)
- forming a pharmaceutically acceptable salt.

Suitably one of R¹³ or R¹⁴ is an activated carboxylic acid derivative, such as an acyl halide or acid anhydride, and the other is an amine group. Activated compounds of formulae (II) or (III) can also be prepared by reaction of the corresponding carboxylic acid with a coupling reagent such as carbonyldiimidazole, dicyclohexylcarbodiimide or diphenylphosphorylazole. Preferably R¹³ or R¹⁴ is a group COL where L is halo, particularly chloro.

A compound of formulae (II) and (III) are typically reacted together in an inert organic solvent such as DMF, THF or dichloromethane at ambient or elevated temperature in the presence of a base such as an alkali metal hydroxide, triethylamine or pyridine.

Intermediate compounds of formulae (II) and (III) are commercially available or can be prepared using standard procedures such as those outlined in EPA 533266/7/8.

It will be appreciated to those skilled in the art that it may be necessary to protect certain reactive substituents during some of the above procedures. Standard protection and deprotection techniques can be used. For example, primary amines can be protected as phthalimide, benzyl, benzyloxycarbonyl or trityl derivatives. These groups can be removed by conventional procedures well known in the art.

Carboxylic acid groups can be protected as esters. Aldehyde or ketone groups can be protected as acetals, ketals, thioacetals or thioketals. Deprotection is achieved using standard conditions.

Certain compounds of formula (I) can be converted into further compounds of formula (I). For example compounds in which R⁷ and R⁸ are both hydrogen or one of R⁷ or R⁸ is hydrogen and the other is C₁₋₆alkyl can be converted to compounds in which R⁷ and R⁸ are both C₁₋₆alkyl using standard alkylation techniques.

5HT_{1D} Antagonists, and in particular the compounds of the present invention, are expected to be of use in the treatment of CNS disorders such as mood disorders, including depression, seasonal effective disorder and dysthymia; anxiety disorders, including generalised anxiety, panic disorder, agoraphobia, social phobia, obsessive compulsive disorder and post-traumatic stress disorder; memory disorders, including dementia, amnestic disorders and age-associated memory impairment; and disorders of eating behaviours, including anorexia nervosa and bulimia nervosa. Other CNS disorders include Parkinson's disease, dementia in Parkinson's disease, neuroleptic-induced Parkinsonism and tardive dyskinesias, as well as other psychiatric disorders.

5HT_{1D} Antagonists, and in particular compounds of the present invention, may also be of use in the treatment of endocrine disorders such as hyperprolactinaemia, in the treatment of vasospasm (particularly in the cerebral vasculature) and hypertension, as well as disorders in the gastrointestinal tract where changes in motility and secretion are involved. They may also be of use in the treatment of sexual dysfunction.

Therefore, the present invention, provides a compound of general formula (I) or a physiologically acceptable salt or solvate thereof for use in therapy.

The present invention also provides a compound of general formula (I) or a physiologically acceptable salt or solvate thereof for use in the treatment of the aforementioned disorders.

In another aspect the invention provides the use of a compound of general formula (I) or a pharmaceutically acceptable salt or solvate thereof for the manufacture of a medicament for the treatment of the aforementioned disorders.

In a further aspect the invention provides a method of treating the aforementioned disorders which comprises administering an effective amount to a patient in need of such treatment of a compound of general formula (I) or a pharmaceutically acceptable salt or solvate thereof.

In particular the invention provides a compound of general formula (I) or a physiologically acceptable salt or solvate thereof for use in the treatment or prophylaxis of depression.

It will be appreciated by those skilled in the art that the compounds according to the invention may advantageously be used in conjunction with one or more other therapeutic agents, for instance, different antidepressant agents.

The present invention also provides a pharmaceutical composition, which comprises a compound of formula (I) or a pharmaceutically acceptable salt thereof, and a pharmaceutically acceptable carrier.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three a day. Such therapy may extend for a number of weeks or months.

The following Examples illustrate the preparation of compounds of the invention.

### Description 1

### 2-(Dimethylaminoethoxy)-4-nitroanisole

A stirred solution of 2-methoxy-5-nitrophenol (5.0g, 0.029 mole) and potassium carbonate (8.3g, 0.060 mole) in acetone (200 ml) and water (60 ml) was treated with N,N-dimethylaminoethyl chloride hydrochloride (8.64g, 0.060 mole) and heated under reflux for 10 h. The mixture was concentrated under vacuum to approx. 80 ml volume, then acidified with 2M HCl acid (150 ml) and washed with ethyl acetate (2 x 80 ml). The acid solution was basified with K₂CO₃ and extracted with ethyl acetate (2 x 100 ml). The combined extract was dried (Na₂SO₄) and concentrated under vacuum to afford the title compound as a yellow solid (4.87g, 70%).
¹H NMR (250 MHz) CDCl₃
- δ:: 7.92 (1H, dd), 7.77 (1H, d), 6.91 (1H, d), 4.18 (2H, t), 3.96 (3H, s), 2.82 (2H, t), 2.37 (6H, s).

### Description 2

### 2-(Dimethylaminoethoxy)4-methoxyaniline

A solution of 2-(dimethylaminoethoxy)-4-nitroanisole (D1, 4.8g, 0.020 mole) in ethanol (200 ml) was hydrogenated over 10% Pd-C (0.5g) at room temperature and pressure. When reduction was complete (lh), the catalyst was removed by filtration through kieselguhr and the filtrate concentrated under vacuum to afford the title compound as a pink solid (4.0g, 95%).
¹H NMR (250 MHz) CDCl₃
- δ:: 6.71 (1H, d), 6.33 (1H, d), 6.24 (1H, dd), 4.07 (2H, t), 3.78 (3H, s), 3.46 (2H, br s), 2.76 (2H, t), 2.33 (6H, s)

### Description 3

### 4'-Methoxycarbonyl-2'-methylbiphenyl-4-carboxylic acid

A stirred solution of methyl 4-bromo-3-methylbenzoate (EP 0533268 A1) (1.0g, 0.0044 mole) in dry DMF (10ml) under argon was treated with 4-boronobenzoic acid (0.73g, 0.0044 mole) and tetrakis (triphenylphosphine)palladium(0) (80mg), followed by triethylamine (1.8ml, 0.016 mole). The mixture was heated at 100°C for 18 hours, then concentrated *in vacuo.* The residue was treated with ethyl acetate and extracted with 10% NaHCO₃ solution. The basic extract was acidified with dil. HCI and extracted with ethyl acetate. The extract was dried (Na₂SO₄) and concentrated *in vacuo* to afford the title compound as a white solid (0.46g, 39%).
¹H NMR (250MHz, d⁶DMSO)
δ(ppm): 13.1 (brs, 1H), 8.04 (d, 2H), 7.93 (s, 1H), 7.87 (d, 1H), 7.51 (d, 2H), 7.38 (d,lH), 3.87 (s, 3H), 2.30 (s, 3H)

### Description 4

### 4'-Carboxamido-2'-methylbiphenyl-4-carboxylic acid

4-Bromo-3-methylbenzamide (0.369g, 1.725mmole) was suspended in 1,2-dimethoxyethane (20ml) and was treated with 4-carboxyphenylboronic acid (0.286g, 1.725mmole), followed by a solution of sodium carbonate (0.823g, 7.76mmole) in water (20ml). The mixture was flushed with argon and tetrakistriphenylphosphinepalladium (0) (0.040g) was added. The reaction mixture was then heated to reflux with stirring. After 24h, the 1,2-dimethoxyethane was removed by evaporation under reduced pressure and the aqueous residue was extracted with ethyl acetate. The aqueous layer was then acidified to pH1 and the resultant solid was filtered off and was dried *in vacuo* to give the title compound as a white solid (0.393g, 89%).
¹H NMR (250MHz, d⁶DMSO)
δ(ppm): 13.0 (brs, 1H), 8.02 (d, 3H), 7.82 (s, 1H), 7.78 (d, 1H), 7.52 (d, 2H), 7.38 (s, 1H), 7.28 (d, 1H), 2.38 (s, 3H)

### Description 5

### N-Methyl-4'-carboxamido-2'-methylbiphenyl-4-carboxylic acid

Using the method outlined in Description 4, N-methyl-4-bromo-3-methylbenzamide (0.363g, 1.59mmole) was converted to the title compound as a white solid (0.338g, 79%)
¹H NMR (250MHz, d⁶DMSO)
δ(ppm): 8.52 (dd, 1H), 8.02 (d, 2H), 7.80 (s, 1H), 7.75 (d, 1H), 7.50 (d, 2H), 7.30 (d, 1H), 2.80 (d, 3H), 2.30 (s, 3H)

### Description 6

### N, N-Dimethyl-4'-carboxamido-2'-methylbiphenyl-4-carboxylic acid

Using the method outlined in Description 4, N, N-dimethyl-4-bromo-3-methylbenzamide (0.383g, 1.583mmole) was converted to the title compound as a white solid (0,354g, 79%)
¹H NMR (250MHz, d⁶DMSO)
δ(ppm): 8.02 (d, 2H), 7.52 (d, 2H), 7.38 (s, 1H), 7.29 (s, 2H), 3.00 (s, 6H), 2.30 (s, 3H)

### Description 7

### N-Methoxy-N-methyl-4-bromo-3-methylbenzamide

A stirred suspension of 4-bromo-3-methylbenzoic acid (5.0g, 0.023mole) in thionyl chloride (20ml) was heated under reflux for 2 hours, then concentrated *in vacuo.* The residual acid chloride was dissolved in dichloromethane (100ml) and added dropwise over 10 minutes to a stirred solution of N,O-dimethylhydroxylamine hydrochloride (2.4g, 0.025mole) and pyridine (5.6ml, 0.069mole) in dichloromethane (150ml) and acetonitrile (20ml) at -20°C. The reaction mixture was allowed to warm to room temperature over 3 hours then treated with 10% Na₂CO₃ solution and extracted with dichloromethane. The extract was dried (Na₂SO₄) and concentrated *in vacuo* to afford the title compound as a pale yellow oil (5.9g, 100%).
¹H NMR (200MHz, CDCl₃)
δ (ppm):7.60-7.50 (m, 2H), 7.37 (dd, 1H), 3.54 (s, 3H), 3.35 (s, 3H), 2.42 (s, 3H)

### Description 8

### 4-Bromo-3-methylacetophenone

A solution of N-methoxy-N-methyl-4-bromo-3-methylbenzamide (D7) (1.50g, 0.0057mole) in dry ether (30ml) was added dropwise over 10 minutes to a stirred solution of methylmagnesium iodide (0.0075mole) in dry ether (15ml) under argon. The mixture was then heated under reflux for 1 hour, allowed to cool and poured into well stirred 1M HCI (50ml). The mixture was extracted with ethyl acetate and the extract washed with 10% Na₂CO₃ solution, dried (Na₂SO₄) and concentrated *in vacuo* to afford the title compound as a pale yellow oil (1.14g, 94%).
¹H NMR (250MHz, CDCl₃)
δ (ppm):7.81 (s, 1H), 7.62 (s, 2H), 2.57 (s, 3H), 2.45 (s, 3H)

### Description 9

### 4'-Acetyl-2'-methylbiphenyl-4-carboxylic acid

The title compound was prepared from 4-bromo-3-methylacetophenone (D8) using a procedure similar to Description 4 (80%).
¹H NMR (250MHz, CDCl₃)
δ (ppm):8.13 (d, 2H), 7.88 (d, 1H), 7.84 (d, 1H), 7.40 (d, 2H), 7.34 (d, 1H), 2.65 (s, 3H), 2.34 (s, 3H)

### Example 1

### N-[3-(2-Dimethylaminoethoxy)4-methoxyphenyl]-2'-methyl-4'-cyanobiphenyl-4-carboxamide

The product from Description 4 (0.100g, 0.392mmole) was suspended in thionyl chloride (7ml) and heated to reflux. After 0.5h, the reaction mixture was allowed to cool and was evaporated under reduced pressure. The yellow oily residue was then azeotroped with toluene (1×10ml) and was dried *in vacuo* to give the crude acid chloride as a yellow solid. The product from Description 2 was dissolved in dry THF (2ml), a solution of sodium hydroxide (0.048g, 1.18mmole) in water (2ml) was added and to this stirred mixture was added the crude acid chloride in THF (2ml). The mixture was then stirred at room temperature for 2h before being partitioned between CH₂Cl₂ and water. The aqueous layer was then extracted with CH₂Cl₂ and the combined organic layers were dried (Na₂SO₄) and evaporated under reduced pressure to give a buff solid. This was purified by preparative T.L.C. using 10% MeOH/CH₂Cl₂ as eluant to give the title compound as a colourless oil (0.046g, 27%) which was converted to its oxalate salt mp 207-208°C
¹H NMR (270MHz, d⁶DMSO)
δ(ppm): 10.30 (s, 1H), 8.04 (d, 2H), 7.88 (s, 1H), 7.78 (d, 1H), 7.62 (s, 1H), 7.54 (d, 2H), 7.44 (d, 1H), 7.39 (d, 1H), 7.00 (d, 1H), 4.28 (t, 2H), 3.89 (s, 3H), 3.42 (t, 2H), 2.84 (s, 6H), 2.32 (s, 3H)

### Example 2

### N-[3-(2-Dimethylaminoethoxy)-4-methoxyphenyl]-4'-methoxycarbonyl-2'-methylbiphenyl-4-carboxamide

The title compound was prepared from 4'-methoxycarbonyl-2'-methylbiphenyl-4-carboxylic acid (D3) and 2-(dimethylaminoethoxy)-4-methoxyaniline (D2) using a similar procedure to Example 1 as a white solid (13%) mp 131-133°C.
¹H NMR (250MHz, CDCl₃)
δ(ppm): 8.3 (brs, 1H), 8.03-7.87 (m, 4H), 7.51 (d, 1H), 7.40 (d, 2H), 7.28 (d, 1H), 7.11 (dd, 1H), 6.84 (d, 1H), 4.12 (t, 2H), 3.94 (s, 3H), 3.84 (s,3H), 2.78 (t, 2H), 2.30 (s, 9H).

### Example 3

### N-[3-(2-Dimethylaminoethoxy)-4-methoxyphenyl]-2'-methyl-4'-N-methylcarboxamidobiphenyl-4-carboxamide

The product from Description 5 (0.170g, 0.632mmole) was suspended in dichloromethane (10ml) and treated with oxalyl chloride (0.083ml, 0.948mmole) followed by a drop of dry DMF. The mixture was then stirred at room temperature for 16h, before being evaporated under reduced pressure and dried *in vacuo.* The resulting pale yellow solid was then redissolved in dichloromethane (5ml) and added to a stirred solution of the product from Description 2 (0.126g, 0.600mmole) in dichloromethane (10ml) containing triethylamine (0.083ml, 0.600mmole). The reaction mixture was then stirred at room temperature overnight before being washed with sodium bicarbonate solution. The organic layer was then dried (Na₂SO₄) and was evaporated under reduced pressure to give a brown oil, which was purified by silica-gel chromatography (10% MeOH/CH₂Cl₂ as eluant) to give the title compound as a pale yellow oil (0.066g, 23%) which was converted to its oxalate salt mp 208-210°C
¹H NMR free base (250MHz, CDCl₃)
δ(ppm): 8.03 (s, 1H), 7.92 (d, 2H), 7.71 (s, 1H), 7.62 (d, 1H), 7.51 (s, 1H), 7.39 (d, 2H), 7.21 (s, 1H), 7.11 (dd, 1H), 6.88 (d, 1H), 6.29 (q, 1H), 4.20 (t, 2H), 3.83 (s, 3H), 3.08 (d, 3H), 2.82 (t, 2H), 2.40 (s, 6H), 2.30 (s, 3H)

### Example 4

### N-[3-(2-Dimethylaminoethoxy)-4-methoxyphenyl)-2'-methyl-4'-N,N-dimethylcarboxamidobiphenyl-4-carboxamide

Following the method outlined in Example 3, the product from description 6(0.150g, 0.530mmole) was transformed into the title compound (0.097g) which was converted to its oxalate salt mp 181-183°C
¹H NMR free base (250MHz, CDCl₃)
δ(ppm): 8.41 (s, 1H), 7.97 (d, 2H), 7.58 (s, 1H), 7.38 (s, 1H), 7.32-7.14 (m, 5H), 6.87 (d, 1H), 4.30 (t, 2H), 3.89 (s, 3H), 3.18 (s, 3H), 3.04 (s, 3H), 2.84 (t, 2H), 2.41 (s, 6H), 2.32 (s, 3H)

### Example 5

### N-[3-(2-Dimethylaminoethoxy)-4-methoxyphenyl]-2'-methyl-4'-carboxamidobiphenyl-4-carboxamide

The product from Description 4 (0.113g, 0.551mmole), and the product from Description 2 (0.116g, 0.551 mmole) were dissolved together in dry DMF (5ml) and EDC.HCl (0.111g, 0.57mmole) was added. The mixture was then stirred at room temperature. After 16h, the reaction mixture was evaporated under reduced pressure to give a brown oil. Water (20ml) was added and the resultant solid filtered off and dried *in vacuo,* before being recrystallised from methanol to give the title compound as an off white solid (0.013g, 5%) mp 212-213°C
¹H NMR (270MHz, d⁶DMSO)
δ(ppm): 10.18 (s, 1H), 8.04 (d, 3H), 7.90 (s, 1H), 7.78 (d, 1H), 7.52 (d, 3H), 7.33 (m, 3H), 6.95 (d, 1H), 4.04 (t, 2H), 3.79 (s, 3H), 2.64 (t, 2H), 2.30 (s, 3H), 2.20 (s, 6H)

### Example 6

### N-[3-(2-Dimethylaminoethoxy)-4-methoxyphenyl]-4'-acetyl-2'-methylbiphenyl-4-carboxamide

The title compound was prepared from 4'-acetyl-2'-methylbiphenyl-4-carboxylic acid (D9) followed a similar procedure to Example 3 (60%) mp 129-130°C.
¹H NMR (250MHz, CDCl₃)
δ (ppm): 8.00-7.82 (m, 5H), 7.48 (d, 1H), 7.43 (d, 2H), 7.32 (d, 1H), 7.08 (dd, 1H), 6.86 (d, 1H), 4.16 (t, 2H), 3.85 (s, 3H), 2.80 (t, 2H), 2.65 (s, 3H), 2.34 (s, 6H), 2.33 (s, 3H)

### Example 7

### N-[3-(2-Dimethylaminoethoxy)-4-methoxyphenyl]-4'-[1-(methoxyimino)ethyl]-2'-methylbiphenyl-4-carboxamide

Methoxylamine hydrochloride (45mg, 0.54 mmole) was added to a stirred solution of potassium t-butoxide (45mg, 0.40mmole) in methanol (5ml) under argon. After 20 minutes at room temperature, the solution was treated with a solution of N-[3-(2-dimethylaminoethoxy)-4-methoxyphenyl]-4'-actyl-2'-methylbiphenyl-4-carboxamide (E6, 120mg, 0.27 mmole) in methanol (3ml) and stirred at room temperature for 18 hours, followed by 1 hour heating under reflux. The solution was allowed to cool, treated with 10% Na₂CO₃ solution (40ml) and extracted with ethyl acetate. The extract was dried (Na₂SO₄), concentrated *in vacuo* and the residue recrystallised from ethyl acetate /60-80 petrol to afford the title compound as a white solid (38mg, 30%) mp 165-167°C.
¹H NMR (250MHz, CDCl₃)
δ (ppm): 7.98 (s, 1H), 7,92 (d, 2H), 7.59 (d, 1H), 7.56-7.47 (m, 2H), 7.41 (d, 2H), 7.26 (d, 1H), 7.08 (dd, 1H), 6.84 (d, 1H), 4.14 (t, 2H), 4.02 (s, 3H), 3.85 (s, 3H), 2.79 (t, 2H), 2.32 (s, 6H), 2.30 (s, 3H), 2.26 (s, 3H)

### Example 8

### N-[3-(2-Dimethylaminoethoxy)-4-methoxyphenyl]-4'-[1-(hydroxyimino)ethyl]-2'-methylbiphenyl-4-carboxamide

The title compound was prepared from hydroxylamine hydrochloride and N-[3-(2-dimethylaminoethoxy)-4-methoxyphenyl]-4'-acetyl-2'-methylbiphenyl-4-carboxamide (E6) using a similar procedure to Example 7 (34%) mp 219-220°C
¹H NMR (200MHz, d⁶ DMSO)
δ (ppm): 11.25 (s, 1H), 10.15 (s, 1H), 8.03 (d, 2H), 7.65-7.47 (m, 5H), 7.37 (dd, 1H), 7.27 (d, 1H), 6.95 (d, 1H), 4.13 (t, 2H), 3.75 (s, 3H), 2.66 (t, 2H), 2.30 (s, 3H), 2.23 (s, 6H), 2.19 (s, 3H)

## Claims

1. A compound of formula (I) or a salt thereof: 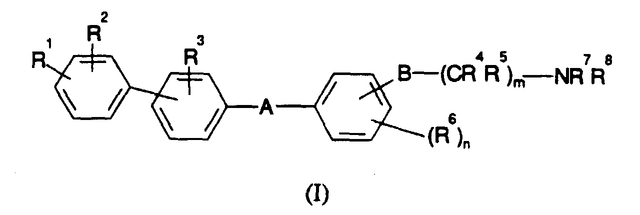 in which
R¹ is hydrogen, halogen, C₁₋₆alkyl, C₃₋₆cycloalkyl, COC₁₋₆alkyl, C₁₋₆alkoxy, hydroxy, hydroxyC₁₋₆alkyl, hydroxyC₁₋₆alkoxy, C₁₋₆alkoxyC₁₋₆-alkoxy, acyl, nitro, trifluoromethyl, cyano, SR⁹, SOR⁹, SO₂R⁹, SO₂NR¹⁰R¹¹ CO₂R¹⁰, CONR¹⁰R¹¹, CO₂NR¹⁰R¹¹, CONR¹⁰(CH₂)ₚCO₂R¹¹, (CH₂)ₚNR¹⁰R¹¹, (CH₂)ₚCONR¹⁰R¹¹, (CH₂)ₚCO₂C₁₋₆alkyl, NR¹⁰R¹¹, NR¹⁰CO₂R¹¹, NR¹⁰CONR¹⁰R¹¹, CR¹⁰=NOR¹¹, CNR¹⁰=NOR¹¹, where R¹⁰ and R¹¹ are independently hydrogen or C₁₋₆alkyl and p is 1 to 3;
R² and R³ are independently hydrogen, halogen, C₁₋₆alkyl, C₃₋₆cycloalkyl, C₃₋₆cycloalkenyl, C₁₋₆alkoxy, acyl, aryl, acyloxy, hydroxy, nitro, trifluoromethyl, cyano, CO₂R¹⁰, CONR¹⁰R¹¹, NR¹⁰R¹¹ where R¹⁰ and R¹¹ are independently hydrogen or C₁₋₆alkyl;
R⁴ and R⁵ are independently hydrogen or C₁₋₆alkyl;
R⁶ is hydrogen, halogen, hydroxy, C₁₋₆alkyl or C₁₋₆alkoxy;
R⁷ and R⁸ are independently hydrogen, C₁₋₆alkyl, aralkyl, or together with the nitrogen atom to which they are attached form an optionally substituted 5-7-membered heterocyclic ring containing one or two heteroatoms selected from oxygen, nitrogen or sulphur;
A is CONH or NHCO;
B is oxygen, S(O)p where p is 0, 1 or 2, NR¹² where R¹² is hydrogen,
C₁₋₆alkyl or phenylC₁₋₆alkyl, or B is CR⁴=CR⁵ where R⁴ and R⁵ are independently hydrogen or C₁₋₆alkyl;
m is 1 to 4; and
n is 1 or 2.

2. A compound according to claim 1 in which R¹ is cyano, acyl, CO₂R¹⁰, CONR¹⁰R¹¹ or CR¹⁰=NOR¹¹.

3. A compound according to claim 2 or 3 in which R² is C₁₋₆alkyl.

4. A compound according to any one of claims 1 to 3 in which R³ is hydrogen

5. A compound according to any one of claims 1 to 4 in which B is oxygen.

6. A compound according to any one of claims 1 to 5 in which m is 1 or 2 and R⁴ and R⁵ are both C₁₋₆alkyl.

7. A compound according to claim 1 which is:
N-[3-(2-Dimethylaminoethoxy)-4-methoxyphenyl]-4'-methoxycarbonyl-2'-methylbiphenyl-4-carboxamide,
N-[3-(2-Dimethylaminoethoxy)-4-methoxyphenyl]-2'-methyl-4'-N-methylcarboxamidobiphenyl-4-carboxamide,
N-[3-(2-Dimethylaminoethoxy)-4-methoxyphenyl]-2'-methyl-4'-N,N-dimethylcarboxamidobiphenyl-4-carboxamide,
N-[3-(2-Dimethylaminoethoxy)-4-methoxyphenyl]-2'-methyl-4'-carboxamidobiphenyl-4-carboxamide,
N-[3-(2-Dimethylaminoethoxy)-4-methoxyphenyl]-2'-methyl-4'-cyanobiphenyl-4-carboxamide,
N-[3-(2-Dimethylaminoethoxy)-4-methoxyphenyl]-4'-acetyl-2'-methylbiphenyl-4-carboxamide,
N-[3-(2-Dimethylaminoethoxy)-4-methoxyphenyl]-4'-[1-(methoxyimino)ethyl]-2'-methylbiphenyl-4-carboxamide,
N-[3-(2-Dimethylaminoethoxy)-4-methoxyphenyl]-4'-[1-(hydroxyimino)ethyl]-2'-methylbiphenyl-4-carboxamide,
or a pharmaceutically acceptable salt thereof.

8. A process for the preparation of a compound of formula (I) which comprises
(a) reaction of a compound of formula (II): 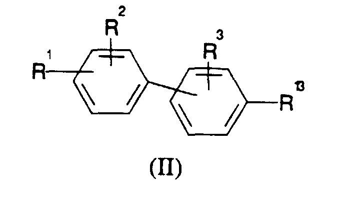 with a compound of formula (III): 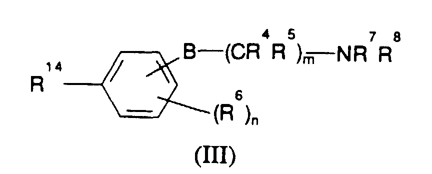 wherein B, m, n, R¹, R², R³, R⁴, R⁵, R⁶, R⁷ and R⁸ are as defined in formula (I) and R¹³ and R¹⁴ contain the appropriate functional group(s) necessary to form the A moiety; and optionally thereafter in any order:
• converting a compound of formula (I) into another compound of formula (I)
• forming a pharmaceutically acceptable salt.

9. A compound according to any one of claims 1 to 7 for use in therapy.

10. A pharmaceutical composition which comprises a compound according to any one of claims 1 to 7 in association with a pharmaceutically acceptable carrier or excipient.

## Patentansprüche

1. Verbindung der Formel (I) oder ein Salz davon 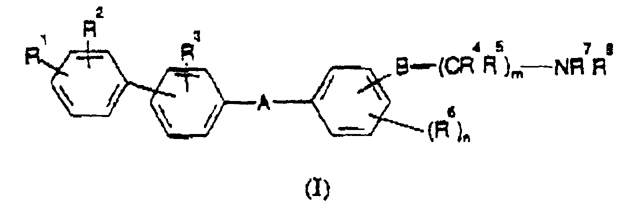 in der
R¹ ein Wasserstoffatom, ein Halogenatom, ein C₁₋₆-Alkyl-, C₃₋₆-Cycloalkyl-, COC₁₋₆-Alkyl -, C₁₋₆-Alkoxyrest, eine Hydroxygruppe, ein Hydroxy-C₁₋₆-alkyl-, Hydroxy-C₁₋₆-alkoxy-, C₁₋₆-Alkoxyrest, eine Acyl-, Nitro-, Trifluormethyl-, Cyanogruppe, SR⁹, SOR⁹, SO₂R⁹, SO₂NR¹⁰R¹¹, CO₂R¹⁰, CONR¹⁰R¹¹, CO₂NR¹⁰R¹¹, CONR¹⁰(CH₂)ₚCO₂R¹¹, (CH₂)ₚNR¹⁰R¹¹, (CH₂)ₚCONR¹⁰R¹¹, ein (CH₂)ₚCO₂C₁₋₆-alkylrest, NR¹⁰R¹¹, NR¹⁰CO₂R¹¹, NR¹⁰CONR¹⁰R¹¹, CR¹⁰=NOR¹¹, CNR¹⁰=NOR¹¹ ist, wobei R¹⁰ und R¹¹ unabhängig voneinander ein Wasserstoffatom oder einen C₁₋6-Alkylrest bedeuten und p 1 bis 3 ist;
R² und R³ unabhängig voneinander ein Wasserstoffatom, ein Halogenatom, einen C₁₋₆-Alkyl-, C₃₋₆-Cycloalkyl-, C₃₋₆-Cycloalkenyl-, C₁₋₆-Alkoxy-, Acyl-, Aryl-, Acyloxyrest, eine Hydroxy-, Nitro-, Trifluormethyl-, Cyanogruppe, CO₂R¹⁰, CONR¹⁰R¹¹, NR¹⁰R¹¹ sind, wobei R¹⁰ und R¹¹ unabhängig voneinander ein Wasserstoffatom oder einen C₁₋₆-Alkylrest bedeuten;
R⁴ und R⁵ unabhängig voneinander ein Wasserstoffatom oder ein C₁₋₆-Alkylrest sind;
R⁶ ein Wasserstoffatom, ein Halogenatom, eine Hydroxygruppe, ein C₁₋₆-Alkyl- oder C₁₋₆-Alkoxyrest ist;
R⁷ und R⁸ unabhangig voneinander ein Wasserstoffatom, ein C₁₋₆-Alkyl-, Aralkylrest sind, oder zusammen mit dem Stickstoffatom, an das sie gebunden sind, einen wahlweise substituierten 5- bis 7-gliedrigen heterocyclischen Ring bilden, der ein oder zwei Heteroatome, ausgewählt aus Sauerstoff, Stickstoff oder Schwefel, enthält;
A CONH oder NHCO ist;
B Sauerstoff, S(O)ₚ, wobei p 0, 1 oder 2 ist, NR¹², wobei R¹² ein Wasserstoffatom ist, ein C₁₋₆-Alkyl- oder Phenyl-C₁₋₆-alkylrest ist, oder B CR⁴=CR⁵ ist, wobei R⁴ und R⁵ unabhängig voneinander ein Wasserstoffatom oder einen C₁₋₆-Alkylrest bedeuten;
m 1 bis 4 ist; und
n 1 oder 2 ist.

2. Verbindung nach Anspruch 1, in der R¹ eine Cyanogruppe, ein Acylrest, CO₂R¹⁰, CONR¹⁰R¹¹ oder CR¹⁰=NOR¹¹ ist.

3. Verbindung nach Anspruch 2 oder 3, in der R² ein C₁₋₆-Alkylrest ist.

4. Verbindung nach einem der Ansprüche 1 bis 3, in der R³ ein Wasserstoffatom ist.

5. Verbindung nach einem der Ansprüche 1 bis 4, in der B ein Sauerstoffatom ist.

6. Verbindung nach einem der Ansprüche 1 bis 5, in der m 1 oder 2 ist, und R⁴ und R⁵ beide ein C₁₋₆-Alkylrest sind.

7. Verbindung nach Anspruch 1, die
N-[3-(2-Dimethylaminoethoxy)-4-methoxyphenyl]-4'-methoxycarbonyl-2'-methylbiphenyl-4-carboxamid,
N-[3-(2-Dimethylaminoethoxy)-4-methoxyphenyl] -2'-methyl-4'-N-methylcarboxamidobiphenyl-4-carboxamid,
N-[3-(2-Dimethylaminoethoxy)-4-methoxyphenyl]-2'methyl-4'-N,N-dimethylcarboxamidobiphenyl-4-carboxamid,
N-[3-(2-Dimethylaminoethoxy)-4-methoxyphenyl]-2'-methyl-4'-carboxamidobiphenyl-4-carboxamide,
N-[3-(2-Ditnethylaminoethoxy)-4-methoxyphenyl]-2'-methyl-4'-cyanobiphezyl-4-carboxamid;
N-[3-(2-Dimethylaminoethoxy)-4-methoxyphenyl]-4'-acetyl-2'-methylbiphenyl-4-carboxamid,
N-[3-(2-Dimethylaminoethoxy)-4-methoxyphenyl]-4'-[1-(methoxyimino)ethyl]-2'-methylbiphenyl-4-carboxamid,
N-[3-(2-Dimethylaminoethoxy)-4-methoxyphenyl]-4'-[1-(hydroxyimino)ethyl]-2'-methylbiphenyl-4-carboxamid,
oder ein pharmazeutisch verträgliches Salz davon ist.

8. Verfahren zur Herstellung einer Verbindung der Formel (I), umfassend
(a) Umsetzen einer Verbindung der Formel (II) 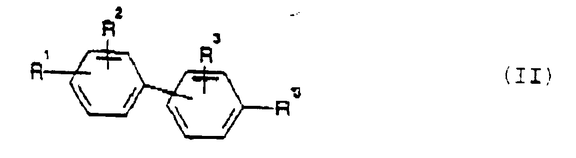 mit einer Verbindung der Formel (III) 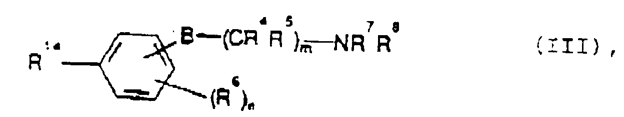 in der B, m, n, R¹, R² R³, R⁴, R⁵, R⁶, R⁷ und R⁸ die in Formel (I) angegebenen Bedeutungen haben, und R¹³ und R¹⁴ eine oder mehrere geeignete funktionelle Gruppe(n) enthalten, die notwendig sind, den Rest A zu bilden;
und wahlweise danach in beliebiger Reihenfolge
- Umwandeln einer Verbindung der Formel (I) in eine andere Verbindung der Formel (I)
- Ausbilden eines pharmazeutisch verträglichen Salzes.

9. Verbindung nach einem der Ansprüche 1 bis 7 zur therapeutischen Verwendung.

10. Pharmazeutische Zusammensetzung, die eine Verbindung nach einem der Ansprüche 1 bis 7 zusammen mit einem pharmazeutisch verträglichen Träger oder Arzneimittelträger umfaßt.

## Revendications

1. Composé de formule (I) ou un de ses sels : 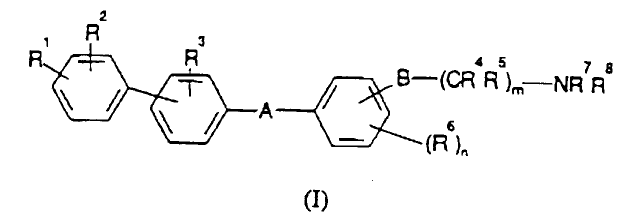 formule dans laquelle
R¹ représente l'hydrogène, un halogène, un groupe alkyle en C₁ à C₆, cycloalkyle en C₃ à C₆, COC-(alkyle en C₁ à C₆), alkoxy en C₁ à C₆, hydroxy, hydroxyalkyle en C₁ à C₆, hydroxyalkoxy en C₁ à C₆, (alkoxy en C₁ à C₆)-(alkoxy en C₁ à C₆), acyle, nitro, trifluorométhyle, cyano, SR⁹, SOR⁹, SO₂R⁹, SO₂NR¹⁰R¹¹, CO₂R¹⁰, CONR¹⁰R¹¹, CO₂NR¹⁰R¹¹, CONR¹⁰(CH₂)ₚCO₂R¹¹, (CH₂)ₚNR¹⁰R¹¹, (CH₂)ₚCONR¹⁰R¹¹, (CH₂)ₚCO₂(alkyle en C₁ à C₆), NR¹⁰R¹¹, NR¹⁰CO₂R¹¹, NR¹⁰CONR¹⁰R¹¹, CR¹⁰=NOR¹¹ ou CNR¹⁰=NOR¹¹, dans lequel R¹⁰ et R¹¹ représentent, indépendamment, l'hydrogène ou un groupe alkyle en C₁ à C₆ et p a une valeur de 1 à 3 ;
R² et R³ représentent, indépendamment, l'hydrogène, un halogène, un groupe alkyle en C₁ à C₆, cycloalkyle en C₃ à C₆, cycloalcényle en C₃ à C₆, alkoxy en C₁ à C₆, acyle, aryle, acyloxy, hydroxy, nitro, trifluorométhyle, cyano, CO₂R¹⁰, CONR¹⁰R¹¹ ou NR¹⁰R¹¹ dans lequel R¹⁰ et R¹¹ représentent, indépendamment, l'hydrogène ou un groupe alkyle en C₁ à C₆ ;
R⁴ et R⁵ représentent, indépendamment, l'hydrogène ou un groupe alkyle en C₁ à C₆ ;
R⁶ représente l'hydrogène, un halogène, un groupe hydroxy, alkyle en C₁ à C₆ ou alkoxy en C₁ à C₆ ;
R⁷ et R⁸ représentent, indépendamment, l'hydrogène, un groupe alkyle en C₁ à C₆, un groupe aralkyle ou, conjointement avec l'atome d'azote auquel ils sont fixés, forment un noyau hétérocyclique penta- à heptagonal facultativement substitué contenant un ou deux hétéroatomes choisis entre l'oxygène, l'azote et le soufre ;
A représente un groupe CONH ou un groupe NHCO ;
B représente l'oxygène, un groupe S(O)ₚ dans lequel p est égal à 0, 1 ou 2, NR¹² dans lequel R¹² représente l'hydrogène, un groupe alkyle en C₁ à C₆ ou phényl(alkyle en C₁ à C₆), ou bien B représente un groupe CR⁴=CR⁵ dans lequel R⁴ et R⁵ représentent, indépendamment, l'hydrogène ou un groupe alkyle en C₁ à C₆ ;
m a une valeur de 1 à 4 ; et
n est égal à 1 ou 2.

2. Composé suivant la revendication 1, dans lequel R¹ représente un groupe cyano, acyle, CO₂R¹⁰, CONR¹⁰R¹¹ ou CR¹⁰=NOR¹¹.

3. Composé suivant la revendication 2 ou 3, dans lequel R² représente un groupe alkyle en C₁ à C₆.

4. Composé suivant l'une quelconque des revendications 1 à 3, dans lequel R³ représente l'hydrogène.

5. Composé suivant l'une quelconque des revendications 1 à 4, dans lequel B représente l'oxygène.

6. Composé suivant l'une quelconque des revendications 1 à 5, dans lequel m est égal à 1 ou 2 et R⁴ et R⁵ représentent l'un et l'autre un groupe alkyle en C₁ à C₆.

7. Composé suivant la revendication 1, qui consiste en :
N-[3-(2-diméthylaminoéthoxy)-4-méthoxyphényl]-4'-méthoxycarbonyl-2'-méthylbiphényl-4-carboxamide,
N-[3-(2-diméthylaminoéthoxy)-4-méthoxyphényl]-2'-méthyl-4'-N-méthylcarboxamidobiphényl-4-carboxamide,
N-[3-(2-diméthylaminoéthoxy)-4-méthoxyphényl]-2'-méthyl-4'-N,N-diméthylcarboxamidobiphényl-4-carboxamide,
N-[3-(2-diméthylaminoéthoxy)-4-méthoxyphényl]-2'-méthyl-4'-carboxamidobiphényl-4-carboxamide,
N-[3-(2-diméthylaminoéthoxy)-4-méthoxyphényl]-2'-méthyl-4'-cyanobiphényl-4-carboxamide,
N-[3-(2-diméthylaminoéthoxy)-4-méthoxyphényl]-4'-acétyl-2'-méthylbiphényl-4-carboxamide,
N-[3-(2-diméthylaminoéthoxy)-4-méthoxyphényl]-4'-[1-(méthoxyimino)éthyl]-2'-méthylbiphényl-4-carboxamide,
N-[3-(2-diméthylaminoéthoxy)-4-méthoxyphényl]-4'-[1-(hydroxyimino)éthyl]-2'-méthylbiphényl-4-carboxamide,
ou un de ses sels pharmaceutiquement acceptables.

8. Procédé pour la préparation d'un composé de formule (I), qui comprend
(a) la réaction d'un composé de formule (II) : 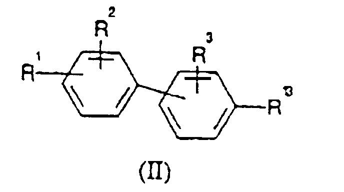 avec un composé de formule (III) : 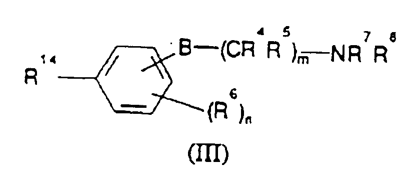 formules dans lesquelles B, m, n, R¹, R², R³, R⁴, R⁵, R⁶, R⁷ et R⁸ répondent aux définitions mentionnées pour la formule (I) et R¹³ et R¹⁴ contiennent le ou les groupes fonctionnels appropriés nécessaires pour la formation du groupement A ; et ensuite, facultativement, dans n'importe quel ordre :
la transformation d'un composé de formule (I) en un autre composé de formule (I),
la formation d'un sel pharmaceutiquement acceptable.

9. Composé suivant l'une quelconque des revendications 1 à 7, destiné à être utilisé en thérapeutique.

10. Composition pharmaceutique qui comprend un composé suivant l'une quelconque des revendications 1 à 7 en association avec un support ou excipient pharmaceutiquement acceptable.
